Europäisches Patentamt

European Patent Office

Office européen des brevets

Publication number: **0 279 175**
**A2**

# EUROPEAN PATENT APPLICATION

Application number: **88100484.0**

Date of filing: **14.01.88**

Int. Cl.4 **A61K 31/07** , A61K 31/19 ,
A61K 31/22

The title of the invention has been amended
(Guidelines for Examination in the EPO, A-III,
7.3).

Priority: **16.01.87 US 4008**

Date of publication of application:
**24.08.88 Bulletin 88/34**

Designated Contracting States:
**AT BE CH DE FR GB IT LI LU NL SE**

Applicant: **THE UNITED STATES OF AMERICA**
**as represented by the Secretary U.S.**
**Department of Commerce**
**National Technical Information Service**
**Office of Government Inventions and**
**Patents 5285 Port Royal Road**
**Springfield, Virginia 22161(US)**

Inventor: **Straw, Gregory Martin**
**9203 Hunting Pines Place**
**Fairfax Virginia 22032(US)**

Representative: **Lederer, Franz, Dr. et al**
**Patentanwält Dr. Franz Lederer Lucile**
**Grahnstrasse 22**
**D-8000 München 80(DE)**

Retinoids in the treatment of psychotic illnesses.

Retinoids, particularly isotretinoin can be used for treating patients having a psychotic illness such as
schizophrenia and/or inhibiting the occurrence of movement disorders in patients receiving or having received a
neuroleptic medication.

EP 0 279 175 A2

## Treatment of psychotic illnesses

The invention relates to the treatment of psychotic illness, particularly schizophrenia, using therapeutic compositions which comprise retinoids, including the retinoic acids, particularly retinoic acid isomers such as 13-cis-retinoic acid and 13-trans-retinoic acid.

Psychotic disorders involve an impairment of mental functioning to the extent that it interferes grossly with an individual's ability to meet the ordinary demands of life, characterized generally by severe affective disturbance, profound introspection, and withdrawal from reality with failure to test and evaluate external reality adequately, formation of delusions or hallucinations, and regression presenting the appearance of personality disintegration. Included in this grouping are the affective disorders, paranoid states and - schizophrenias.

Psychotic disorders may be treated in some cases by the administration of a major tranquilizer, or neuroleptic such as 4-[4-(p-chlorophenyl)-4-hydroxy-piperidino]-4'-fluorobutyrophenone (hereinafter "haloperidol" or "HAL"). The precise mechanism of action of haloperidol has not been clearly established.

Haloperidol generally reaches a steady state blood level after approximately five days of daily dosage and intra-individual variation in blood concentration is limited. Large individual differences in such variables as absorption, distribution, metabolism and excretion, however, result in greater inter-individual differences in concentrations at the same dose. These variables are known to be affected by concomitant administration of other drugs. Commonly used drugs that may affect neuroleptic blood concentrations include lithium, trihexyphenidyl, anticonvulsants, tricyclic and monoamine oxidase inhibitor antidepressants, oral contraceptives, and antacids.

The most often reported effect is for HAL concentrations to decrease with concurrent administration of anticonvulsant drugs such as carbamazepine or phenobarbitol. The exact mechanisms for this effect are not known, but the induction of metabolic enzymes may be the common pathway. A primary metabolite of HAL in humans is the reduced form, 4-(4-(4-chlorophenyl)-4-hydroxy-piperdinyl)-1-(4-fluorophenyl)-1-butanol, hereinafter hydroxy-haloperidol (OH-HAL). However, the actual mechanism for its production in vivo has not been proven. Other possibilities are that the drugs affect the absorption, distribution, metabolism or excretion of haloperidol.

In accordance with the present invention it has been discovered that there is a pharmacokinetic interaction between haloperidol and retinoids, including retinoic acid, which results in decreased serum levels of haloperidol in a patient with litle or no loss of efficacy for the treatment of psychotic illness. Additionally, it has been discovered that the retinoids themselves have a direct psychotropic effect.

Therefore, the present invention relates to a method of treating a patient having a psychotic illness, such as schizophrenia, by the systemic administration of a retinoid to the patient in a pharmaceutically effective amount for treating the psychotic illness. The invention also relates to a method for inhibiting the occurrence of movement disorders, such as tardive dyskinesia and extra-pyramidal side effects resulting from treatment with a neuroleptic such as haloperidol, which method comprises the concurrent administration of a retinoid in a pharmaceutically effective amount for inhibiting the occurrence of the movement disorders.

Retinoids reduce serum levels of neuroleptic medication in patients to whom the retinoids are administered. Moreover, in some patients, the administration of retinoids may, themselves relieve some of the symptoms of psychotic illnesses such as schizophrenia.

One advantage of administering retinoids to a patient afflicted with psychotic illness is that it relieves the patient's symptoms without the dyskinesias or parkinsonium that are associated the the neuroleptic medications such as haloperidol. Moreover, when retinoids are administered to patients who are also receiving one of the commonly adminstered antipsychotic drugs, such as haloperidol, the retinoids ameliorate the dyskinesias and parkinsonium symptoms which might otherwise result from treatment with the antipsychotic.

In accordance with this invention, a pharmaceutical composition comprising an amount of a retinoid which is effective in the treatment of a psychotic illness, such as schizophrenia and a medicinally inert pharmaceutically acceptable carrier material is adminstered to a human suffering from the psychotic illness.

As used herein, "retinoid" denotes the known vitamin A compounds in naturally occuring forms such as retinol, retinal, retinyl esters, retinoic acid as well as the known synthetic analogs of vitamin A. The ring on the analogs may be aromatic or heteroaromatic and the side chain may be optionally substituted with a halide such as chloride. The terminal group may be oxidized, reduced, esterified, etc. The alkali metal (sodium potassium, etc.) and alkaline earth metal (magnesium, calcium, etc.) salts of a retinoic (also called "retinoid carboxylic") acid are also included herein.

Preferred retinoids for practicing the present invention are the various geometric isomers (cis, trans) of retinoic acid, including the alkali metal and alkaline earth metal salts thereof as well as their esters having lower alkyl groups of 1 to 6 carbon atoms.

Particularly preferred retinoic acids for the treatment of psychotic illnesses such as schizophrenia are 13-cis-retinoic acid (isotretinoin) and all-trans-retinoic acid (tretinoin).

In accordance with this invention the retinoids can be administered in a pharmaceutically acceptable dosage form in either a parenteral or enteral mode preferably orally. These pharmaceutical compositions of the invention contain the retinoids as an active ingredient in association with a compatible pharmaceutically acceptable carrier material. Any conventional carrier material can be utilized. The carrier material can be an organic or inorganic inert carrier material suitable for enteral, percutaneous or parenteral administration. Suitable carriers include water, gelatin, gum arabic, lactose, starch, magnesium stearate, talc, vegetable oils, polyalkyleneglycols, petroleum jelly and the like. Furthermore, the pharmaceutical preparations may contain other pharmaceutically active agents. Additional additives such as flavoring agents, preservatives, stabilizers, emulsifying agents, buffers and the like may be added in accordance with accepted practices of pharmaceutical compounding.

The pharmaceutical preparations can be made up in any conventional form including: (a) a solid form for oral administration such as tablets, capsules, pills, dragees, powders, granules, and the like; (b) a liquid form for oral administration such as solutions, syrups, suspensions, elixirs and the like; and (c) preparations for parenteral administration such as sterile solutions, suspensions or emulsions. The pharmaceutical preparations may be sterilized and/or may contain adjuvants such as preservatives, stabilizers, wetting agents, emulsifiers, salts for varying the osmotic pressure and/or buffers.

Parenteral dosage forms may be infusions or injectable solutions which can be injected intravenously or intramuscularly. These preparations can also contain other medicinally active substances. For parenteral formulations a daily dosage of from about 0.01 mg to about 3 mg per Kg of body weight, preferably from about 0.05 mg to about 1 mg per Kg of body weight of the patient most preferably about 0.5 mg per kg of body weight of the patient will be utilized. Additional additives such as flavoring agents, preservatives, stabilizers, emulsifying agents, buffers and the like may be added in accordance with accepted practices of pharmaceutical compounding.

A preferred oral dosage form comprises capsules of hard or soft gelatin methylcellulose or of another suitable material easily dissolved in the digestive tract. The enteral dosages contemplated in accordance with the present invention will vary in accordance with the needs of the individual patient as determined by the prescribing physician. Generally, however, a daily dosage of from about 0.01 mg to about 3 mg per Kg of body weight, preferably from about 0.05 mg to about 1 mg per Kg of body weight of the patient and most preferably about 0.5 mg per Kg of body weight of the patient is utilized. This dosage may be administered according to any dosage schedule determined by the physician in accordance with the requirements of the patient.

It is likewise within the purview of the present invention to incorporate a retinoid in any desired amount for enteral administration within the oral unit dosage form. It is preferred, however, to formulate preparations containing the active retinoid in such a manner that each dose contains from about 0.05 mg to about 100 mg, particularly from about 5 mg to about 40 mg of the active substance with suitable therapeutically inert fillers and diluents. It is especially preferred to incorporate such a dosage into gelatin capsules and tablets.

The following examples illustrate the invention. Examples 1-5 are directed to the preparation of gelatin capsule pharmaceutical preparations containing isotretinoin for oral administration.

EXAMPLE 1

---

### 25% Isotretinoin Suspension

Item Ingredient     grams/Kg

---

1.    Isotretinoin   250.00

2.    Purified Beeswax   36.38

3.    Hydrogenated Soybean Oil Flakes   36.38

      Hydrogenated Vegetable Oil   145.73

4.    Soybean Oil, USP   528.61

5.    Butylated Hydroxyanisole   0.50

6.    Disodium Edetate   2.40

---

Ingredient items 2 through 4 were melted at about 70°C using a suitable heater and mixed using a suitable mixer.

Next, ingredient item 5 was heated to about 38°C using a suitable heater and ingredient item 6 was incorporated and dissolved into ingredient item 5 while mixing with a suitable mixer and maintaining the temperature at about 38°C.

Ingredient items 2 through 6 were then combined while being mixed and were then cooled to about 40°C following mixing.

Isotretinoin was then added with mixing followed by the addition of ingredient item 7 while mixing.

The resulting mixture was cooled to room temperature, this forming the 25% isotretinoin suspension.

In the following examples, the term "wax mixture" shall refer to a mixture of 1 part by weight purified beeswax, 1 part by weight hydrogenated soybean oil flakes and 4 parts by weight hydrogenated vegetable oil.

EXAMPLE 2

| Gelatin Capsule for Oral Administration - 20 mg | |
|---|---|
| 25% Isotretinoin suspension (Example 1) | 84 mg |
| Butylated hydroxyanisole | 0.166 mg |
| Disodium edetate | 0.792 mg |
| Wax mixture | 72.6 mg |
| Soybean oil | 172.442 mg |

The above ingredients were melted and mixed and filled into gelatin capsules.

The retinoids, especially retinoic acid isomers, alone or in combination with another neuroleptic, are effective neuroleptics in humans in the treatment of human psychotic illness, including schizophrenia, and in producing tranquilization. The retinoids, especially retinoic acid isomers are also effective in altering the tissue and serum concentration of concurrently administered neuroleptics such as haloperidol in humans. These effects are demonstrated in the following examples.

The following examples 3-4 show a pharmacokinetic interaction between haloperidol and retinoic acid in humans. In particular, Example 4 also reveals a direct psychotropic effect of retinoic acid.

EXAMPLE 3

A 22 year old male patient (subject 1) was diagonosed as having schizophrenia and was receiving haloperidol at a dose of 0.2 mg/Kg/day in two divided daily doses. His blood level of haloperidol at that time and dose was 13.7 ng/ml (mean of three samples). He was begun subsequently on a concurrent course of isotretinoin at a once daily dose of 0.6 mg/Kg/day orally for the treatment of cystic acne. There was a decrease in the haloperidol blood level following initiation of the isotretinoin to 8.8 ng/ml (1 sample). There was no adverse change in his clinical condition after starting isotretinoin despite the reduction in haloperidol blood levels.

EXAMPLE 4

A 29 year old male patient (subject 2) diagnosed as having schizophrenia was initially maintained in double blind placebo fashion on haloperidol and benztropine for over ten weeks with complaints of persistent auditory hallucinations and poor concentration. His affect was blunted, and he was socially withdrawn. Laboratory evaluation, physical exam, CT head scan, EEG, and EKG were within normal limits.

After his behavioral status as measured by the Brief Psychiatric Rating Scale (BPRS) has stabilized, he was placed on coded active haloperidol at 30 mg/day (0.4mg/Kg/day). The BPRS was performed by nursing staff blind to the haloperidol and benztropine status. He developed parkinsonian symptoms and was placed on benztropine at 4 mg/day increased to 8 mg/day. He clinically improved after being changed from placebo to active haloperidol as reflected by a decrease in his BPRS items. The positive symptoms scale of the BPRS (here called PosBPRS and including hallucinations, delusions, and disorganized thinking) average over the seven days prior to each medication change was 1.83 in his first drug free period, falling over four weeks on haloperidol to 1.00. His serum haloperidol level as measured by high performance liquid chromatography was 19.1 ng/ml. At that time he requested that the dermatologist put him on isotretinoin for his cystic acne because his sibling has recently had a good response. When on isotretinoin he received 40 mg in a single daily dose (0.5 mg/Kg/day). A further improvement in his clinical presentation and PosBPRS (.066) was then seen in spite of a drop over the next 6 weeks of the serum haloperidol blood levels to near zero (<1 ng/ml or unmeasurable on our assay). The serum haloperidol concentration was unmeasurable on four successive samples over the next six weeks in spite of an increase of the haloperidol dose to 60 mg/day (0.8mg/Kg/day). This increase in dose was also followed by a slight further decrease in his PosBPRS scores (0.55).

The isotretinoin then was stopped. Within 2 days the patient became more parkinsonian and more irritable, and began to present ideas of reference and complaints of poorly defined "disturbing thoughts". Two days after stopping isotretinoin his serum haloperidol had risen to 10 ng/ml. The haloperidol, benztropine, and isotretinoin then were discontinued. The haloperidol levels fell to zero within one week. His PosBPRS scores rose to 1.12 over the next three weeks.

The isotretinoin was then restarted without other medications and his PosBPRS scores fell slowly over a six week period to 0.35. The isotretinoin was again stopped. After the isotretinoin was discontinued his clinical presentation worsened with flattening of affect, loss of motivation, and increasing social withdrawal. The deterioration was clinically noticable by three days after withdrawal of the isotretinoin, and his PosBPRS increased to 2.37 after 5 weeks drug free.

Neuropsychological evaluation during the second period of isotretinoin treatment reflected no significant changes in performance levels compared to the mostly above average functioning at the end of the preceding three week drug free period. Exceptions were verbal fluency (improved) and block design (declined). In contrast, neuropsycholgical performance 5 weeks after the second istretinoin course was discontinued showed dramatic declines to below pretreatment levels on measures of simple and complex memory, verbal fluency, attention and cognitive efficiency.

The effect of isotretinoin on haloperidol blood levels is illustrated in Table 1.

## TABLE 1

### EFFECT OF ISOTRETINOIN ON HALOPERIDOL BLOOD LEVELS IN TWO SUBJECTS

| SUBJECT | DATE | HALOPERIDOL SERUM CONCENTRA- TIONS | HALOPERI- DOL DOSE (MG/DAY) | ISOTRETINOIN DOSE (MG/DAY) | PosBPRS 7 DAY AVE (MG/DAY) |
|---|---|---|---|---|---|
| 1 | 8/13/85 | 13.7 | 14 | 0 | |
| 1 | 12/3/85 | 14.9 | 14 | 0 | |
| 1 | 12/11/85 | 11.6 | 14 | 0 | |
| 1 | 12/17/85 | 10.7 | 14 | 40 | |
| 1 | 12/17/85 | 7.0 | 14 | 40 | |
| 2 | 7/30/85 | 0.0 | 0 | 0 | 1.83 |
| 2 | 8/14/85 | 17.6 | 30 | 0 | |
| 2 | 8/27/85 | 19.1 | 30 | 0 | 1.00 |
| 2 | 9/10/85 | 5.4 | 30 | 40 | |
| 2 | 9/17/85 | 8.6 | 30 | 40 | |
| 2 | 9/23/85 | 3.5 | 30 | 40 | 0.66 |
| 2 | 10/14/85 | 1.0 | 60 | 40 | |
| 2 | 10/21/85 | 1.0 | 60 | 40 | |
| 2 | 11/6/85 | 1.0 | 60 | 40 | 0.55 |
| 2 | 11/19/85 | | | DISC | |
| 2 | 11/22/85 | 1.0 | 60 | 0 | |
| 2 | 11/25/85 | 10.0 | 60 | 0 | |
| 2 | 11/26/85 | | DISC | 0 | |
| 2 | 12/3/85 | 1.0 | 0 | 0 | |
| 2 | 12/9/85 | | | 0 | 1.12 |
| 2 | 12/10/85 | | | 40 | |
| 2 | 2/8/86 | | 0 | 40 | 0.35 |
| 2 | 2/11/86 | | | 0 | |
| 2 | 3/14/86 | | | 9 | 1.57 |

The following examples show animal test data for isotretinoin (Examples 5-6) as well as tretinoin (Example 7).

## EXAMPLE 5

Female Sprague-Dawley rats were obtained from Zivic-Miller Laboratories and weighed 380 g (±s.d.10) when tested.

The HAL and OH-HAL concentrations were measured electrochemically after high performance liquid chromatography (HPLC) in accordance with the method of Korpi et al. (Korpi, E.R., B.H. Phelps, H. Grainger, W. Chang, M. Linnoila, J.L. Meek & R.J. Wyatt: Simultaneous determination of haloperidol and its related metabolite in serum and plasma by isocratic liquid chromatography with electrochemical detection. Clin. Chem., 1983, 29, 624-628). Concentrations were reported in nanograms per milliliter (ng/ml) of spun serum, red blood cells, or homogenized brain tissue. The volumes of the brain samples were calculated from the wet weight of the frozen tissue using the specific gravity measured in control animals at 0.95 g/ml. Overall significance of the effect of isotretinoin ("ISOT") was determined by a repeated measures MANOVA F test with two within-subject variables: tissue compartment and test performed. Significance of the difference between means was then determined at $p < .05$ by the method of Tukey for unbalanced data.

All rats received HAL 1 mg/kg (in 1% lactic acid in physiologic saline injected in a volume of 1 ml/kg) subcutaneously. ISOT was injected intraperitoneally in neat DMSO (at 1 ml/kg) at concentrations to obtain the required dose in mg/kg. DMSO was used to assure complete mobilization of the ISOT in the liquid phase. All ISOT solutions were prepared fresh for each injection series. All work with the ISOT including the injections was done under amber light to avoid photodecomposition of the ISOT.

The animals were marked for identification and the injections were given sequentially at one per month at each session so that each animal received the haloperidol first, followed by the isotretinoin 36 minutes later. There were 35 animals divided into four groups by isotretinoin dose: 0 mg/kg (N = 11), 1 mg/kg (N = 8), 5 mg/kg (N = 8), 10 mg/kg (N = 8). The five injection sessions were done over 3 days beginning at 9 am, 4 pm, 9 am, 4 pm, and 9 am, with the animals then sacrificed by decapitation starting at 1 pm of the third day at one per minute in the same order in which they were injected. Core blood was collected, cooled on ice, spun for 20 minutes at 1000g, and the serum and red cells separated and frozen in plastic tubes at -40 C. until assayed. Within one minute after sacrifice the brain was removed and separated from the brain stem above the pons and placed on ice for approximately 30 minutes. The cerebrum and rostral brain stem were divided in half between the hemispheres and stored at -40 C. One hemisphere and attached rostral brain stem from each animal were weighed, homogenized in double distilled water and an aliquot removed and alkalinized for extraction prior to assay by HPLC.

The concentrations of HAL and OH-HAL were reported in Table 2 for the three tissue compartments: serum, red blood cells and brain. To examine the effect of ISOT on the metabolite/drug relationship, the ratio of OH-HAL to HAL in each compartment was also presented. Thus, there were three tissue compartments each with three test measures evaluated in each animal.

## TABLE 2**

ISOTRETINOIN (mg/kg)

| | 0 | 1 | 5 | 10 |
|---|---|---|---|---|
| Serum | | | | * |
| HAL | 44.8 ±7.1 | 67.5 ±8.0 | 73.3 ±17.1 | 97.5 ±19.0 |
| Serum | | * | · * | |
| OH-HAL | 1.8 ±.2 | 4.2 ±.5 | 3.3 ±.2 | 3.0 ±.4 |
| Serum ratio | | | | |
| OH-HAL/HAL | 0.038 | 0.060 | 0.044 | 0.029 |
| Red blood cells | | | | |
| HAL | 13.4 ±2.4 | 18.1 ±4.8 | 33.8 ±16.8 | 37.2 ±8.8 |
| Red blood cells | | | | |
| OH-HAL | <2 | <2 | <2 | 3.2 ±.8 * |
| Red blood cells ratio | | | * | * |
| OH-HAL/HAL | <.15 | <.11 | <.06 | 0.09 |
| Brain | | | | * |
| HAL | 465 ±44 | 413 ±31 | 344 ±26 | 739 ±90 |
| Brain | | | | |
| OH-HAL | 96 ±13 | 64 ±8 | 45 ±4 | 152 ±28 |
| Brain ratio | | | * | |
| OH-HAL/HAL | 0.216 | 0.15 | 0.13 | 0.19 |

** Table 2. Summarized data for each compartment (mean ±standard error) in nanograms/milliliter (ng/ml). * denotes significant difference from controls at the $p < .05$ level by Tukey's method for multiple comparisons of the means.

The overall between-subject effects of ISOT dose were significant with $F_{(3,31)} = 10.06$, $p = 0.0473$. Within-subject effects for tissue compartment and test were both significant with $F_{(3,62)} = 245.41$, $p = 0.0001$ and $F_{(3,62)} = 396.95$, $p = 0.0001$, respectively. Significant interaction effects were also seen between ISOT dose and both the compartment and the test type with $F_{(6,62)} = 8.95$, $p = 0.0001$ and $F_{(6,62)} = 9.75$, $p = 75$, $p = 0.0001$, respectively.

Serum HAL concentrations were affected significantly with $F_{(3,31)} = 2.96$, $p = 0.0473$. The means for serum HAL tended to increase with each increase in ISOT dose, becoming significantly ($p<.05$) different from controls at the 10 mg/kg level. Red blood cell concentrations of HAL were not significantly altered overall with $F_{(3,31)} = 1.71$, $p = 0.186$. Brain HAL was significantly altered overall with $F_{(3,31)} = 9.75$, $p = 0.0001$. While the brain mean HAL concentration tended to decrease at the intermediate doses, it was significantly increased ($p<.05$) at the 10 mg/kg dose.

The serum OH-HAL concentrations were altered significantly with $F_{(3,31)} = 9.56$, $p = 0.0001$. The means of the serum OH-HAL were significantly increased over controls ($p.<.05$) at the 1 and 5 mg/kg doses of ISOT. Many of the measured OH-HAL values in the red blood cells, particularly at the 1 and 5 mg/kg doses of ISOT, were at or below the minimum sensitivity of the assay reported as <2ng/ml by Korpi et al. (13). For statistical purposes these values were treated as representing primarily the baseline variance of the assay. However, the values were reported here only as their upper limit, <2ng/kg. By this procedure, red blood cell OH-HAL concentrations were significantly altered overall on the repeated measured MANOVA, $F_{(3,31)} = 11.1$, $p = 0.0001$. The mean of the red blood cell OH-HAL concentration at the 10 mg/kg ISOT dose was disignificantly ($p<.05$) elevated above baseline. Brain OH-HAL values were also significantly altered overall, $F_{(3,31)} = 8.27$, $p = 0.0003$, and the individual means tended to follow the pattern of the red cells with a decrease at the low and an increase at the higher dose. However, no individual mean of the brain OH-HAL reached significance.

The ratios of serum OH-HAL to HAL were significantly altered by isotretinoin, $F_{(3,31)} = 3.38$, $p = 0.0306$. However, no mean at any given dose differed significantly from controls. Again for the red blood cell values, the ratios representing the very low values are considered to represent the baseline variance of the measures, and the reported values are truncated at their calculated upper limit. Following this procedure, the ratio in the red blood cells was significantly altered overall, $F_{(3,31)} = 12.66$, $p = 0.0001$, and the separate means were significantly lowered ($p<.05$) at the intermediate doses. The brain ratio of OH-HAL to HAL was significantly changed by ISOT as well, $F_{(3,31)} = 4.86$ at $prob>F = 0.0069$, with only the mean at the 5 mg/kg dose decreasing enough to reach significant ($p<.05$).

Based upon the above data, ISOT when given in DMSO to rats increases the concentrations of HAL and OH-HAL in the serum, brain, and red blood cells. Moreover, there is the appearance at the intermediate ISOT of a decrease in the brain concentrations of both HAL and OH-HAL. This tendency toward a biphasic dose dependent effect of ISOT in the brain and red cells is supported by the finding of a similarly biphasic and significant ($p<.05$) dose dependency in the ratios in these two compartments. Furthermore, the appearance of an opposite biphasic trend in the means of the serum ratios is significant on the repeated measures MANOVA, $F_{(3,31)} = 3.38$, $p = .0306$.

There also are significant compartment-specific variations in the apparent activity of ISOT. The results of the repeated mesures MANOVA indicate that all three of the dependent measures (HAL, OH-HAL, and the ratio OH-HAL/HAL) are affected differently across the three different tissue compartment. The data are consistent with other evidence with the activities of retinoids such as ISOT are tissue specific (Chytil, F.; Retinoic acid; biochemistry, pharmacology, toxicology, and therapeutic use. Pharmacol. Rev., 1984,36,93s-100s).

## EXAMPLE 6

To demonstrate the neuroleptic activity of the retinoic acids in mammals, their tranquilizing effect in mice was tested in the following manner. Female albino laboratory mice from the National Institute of Health stock weighing between 20 to 30 grams were maintained in home cages in groups of less than ten. They were injected intraperitoneally with a suspension of retinoic acid in 10% Tween 80 in physiologic saline and returned to their home cage for two hours. They were then placed individually in an unfamiliar plexiglass chamber where their gross motor activity was automatically counted by two grids of photocells: one for horizontal and one for vertical (rearing) activity. The total count accumulated over 30 minutes in a given orientation (horizontal or vertical) is a measure in arbitrary units of the gross motor exploratory activity of the mouse in this novel environment. Because baseline activity varies from day to day, control values are given for each trial. The results for the intraperitoneal injections are given in Table 3.

0 279 175

## TABLE 3

### MOUSE ACTIVITY HABITUATION
### 2 HOURS AFTER
### 40 MG/KG RETINOIC ACID
### INTRAPERITONEALLY

| ISOMER | HORIZONTAL COUNTS | VERTICAL COUNTS | NUMBER | PROB. (MANOVA) |
|---|---|---|---|---|
| TRETINOIN | $72\pm36$ | $181\pm81$ | 8 | |
| CONTROL | $128\pm48$ | $453\pm244$ | 8 | $p<.02$ |
| ISOTRETINOIN | $117\pm56$ | $371\pm216$ | 4 | |
| CONTROL | $194\pm24$ | $683\pm97$ | 4 | $p<.05$ |

It can be seen from this data that both isomers of retinoic acid cause a similar decrease in spontaneous and exploratory gross motor activity by about 40% in both the vertical and horizontal axis. This is consistent with the fact that there has been demonstrated a naturally occuring enzyme that interconverts these two forms of retinoic acid, and that their activities may be adequately described in some systems as interchangeable.

**Claims**

1. Retinoids for use in the treatment of a psychotic illness and for inhibiting the occurrence of movement disorder side effects of a neuroleptic, such as haloperidol.

2. Retinoids for use in the treatment of schizophrenia.

3. Retinoids for use as in claim 2 when adminstered orally in a daily dose in the range of about 0.01 mg/kg to about 3 mg/kg of the patient's body weight.

4. Isotretinoin for use as in any one of claims 1-3.

5. The use of retinoids in the manufacture of a pharmaceutical composition for the treatment of a psychotic illness and for inhibiting the occurrence of movement disorder side effects of a neuroleptic, such as haloperidol.

6. The use of claim 5 wherein said psychotic illness is schizophrenia.

7. The use of any one of claims 5 and 6 wherein said pharmaceutical composition is for oral application in a unit dosage form, each unit dosage form containing from about 5 mg to about 40 mg of isotretinoin.

8. The use of isotretinoin according to any one of claims 5-7.